# EUROPÄISCHE PATENTSCHRIFT

(11) **EP 2 205 198 B1**
(45) Veröffentlichungstag und Bekanntmachung des Hinweises auf die Patenterteilung: **28.09.2016**
(21) Anmeldenummer: 08847463.0
(22) Anmeldetag: 13.10.2008
(51) Int. Cl.: A61K 8/25, A61K 8/58, A61K 8/891, A61Q 5/06

(54) **STYLINGMITTEL**
STYLING AGENT
AGENT DE STYLISATION

(30) Priorität: 08.11.2007 DE 102007053616
(43) Veröffentlichungstag der Anmeldung: 14.07.2010
(73) Patentinhaber: Henkel AG & Co. KGaA, 40589 Düsseldorf (DE)
(72) Erfinder: RICHTERS, Bernd, 21129 Hamburg (DE); HENTRICH, Dirk, 22457 Hamburg (DE)
(86) Internationale Anmeldenummer: PCT/EP2008/063709
(87) Internationale Veröffentlichungsnummer: WO 2009/059869

(56) Entgegenhaltungen:
- EP-A1- 1 792 640
- WO-A1-2007/051511
- anonymous: "Sun Protective Serum Suncare: Anhydrous gel Formulation 00950", Dow Corning , 27. November 2006 (2006-11-27), Seite 2PP, XP002619944, Gefunden im Internet: URL:http://www.dowcorning.com.cn/zh_CN/con tent/publishedlit/FORMUL_00950.pdf [gefunden am 2011-02-01]
- anonymous: "Dow Corning(R) 9546 Silicone Elastomer Blend", Dow Corning , 11. Februar 2011 (2011-02-11), Seite 8PP, XP002619945, Gefunden im Internet: URL:http://www2.dowcorning.com/DataFiles/0 90007b2815d7cb1.pdf [gefunden am 2011-02-01]
- anonymous: "Soft Solid: Reduced Syneresis, Reduced White Residue FOrmulation 00822", Dow Corning , 1. März 2006 (2006-03-01), Seite 2PP, XP002619946, Gefunden im Internet: URL:http://www.dowcorning.com/content/publ ishedlit/FORMUL_00822.pdf [gefunden am 2011-02-01]
- anonymous: "Cream in Mousse Form", Happi, April 2005 (2005-04), Seite 9PP, XP002619947, Gefunden im Internet: URL:http://www.happi.com/formulary/2005/04 / [gefunden am 2011-02-01]
- ANONYMOUS: 'Dow Corning 1501 Fluid', [Online] 16 September 2011, Seiten 1 - 8, XP055070052 Gefunden im Internet: <URL:http://www3.dowcorning.com/DataFiles/0 90007b28189d538.pdf> [gefunden am 2013-07-08]

## Beschreibung

Die vorliegende Erfindung betrifft die Verwendung kosmetischer Mittel , enthaltend hydrophobiertes Siliziumdioxid, cyclisches Polydimethylsiloxan und ein Siliconelastomer, zur temporären Verformung von Haaren.

Unter keratinischen Fasern werden prinzipiell alle tierischen Haare, z.B. Wolle, Rosshaar, Angorahaar, Pelze, Federn und daraus gefertigte Produkte oder Textilien verstanden. Bevorzugt handelt es sich jedoch um menschliche Haare.

Haarfestigungsmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Solche Mittel werden auch als Stylingmittel bezeichnet, wobei sowohl Haarbehandlungsmittel, die einer permanenten, als auch solche, die einer temporären Formgebung der Haare dienen, eine wichtige Rolle spielen. Temporäre Formgebungen, die einen guten Halt ergeben sollen, ohne das gesunde Aussehen der Haare zu beeinträchtigen, können beispielsweise durch Haarsprays, Haarwachse, Haargele, Fönwellen etc. erzielt werden.

Haarsprays enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Dabei wird in der Regel eine zufrieden stellend gleichmäßige Verteilung des Polymers auf dem Haar erzielt; die Applikation aus Sprüh- oder Pumpbehältern ist leicht und sauber möglich.

Auch Haargele basieren in der Regel auf synthetischen Polymeren. Das Gel wird dabei in Tuben oder in Tiegeln angeboten und wird mit der Hand oder einem geeigneten Hilfsmittel, beispielsweise einem Kamm, auf dem Haar verteilt.

Die wesentliche Anforderung an alle Stylingmittel ist zunächst, dass dem behandelten Haar der gewünschte Halt verliehen wird. Daneben wird es aber zunehmend wichtiger, dass sich das Mittel beim Kontakt mit Haut und Haar angenehm anfühlt, beispielsweise nicht übermäßig klebrig ist. Zudem sind verstärkt Stylingmittel gefragt, die sich durch eine besondere, ungewöhnliche Konsistenz auszeichnen.

Aufgabe der vorliegenden Erfindung war es daher, kosmetische Mittel zur temporären Verformung keratinischer Fasern zur Verfügung zur stellen, die die eben genannten Anforderungen erfüllen.

Es wurde nunmehr gefunden, dass dies durch Kombination bestimmter Siliconkomponenten mit hydrophob derivatisiertem Silica erreicht werden kann. Es lassen sich so Stylingmittel mit einzigartiger Konsistenz erhalten, die auch ohne Zusatz weiterer festigender und/oder filmbildender Polymere den behandelten keratinischen Fasern einen ausgezeichneten Halt verleihen.

Gegenstand der Erfindung sind daher die Verwendung eines kosmetischen Mittels , enthaltend, jeweils bezogen auf das gesamte Mittel,
a) 2 bis 15 Gew.-% mindestens eines hydrophobierten Siliziumdioxids,
b) 50 bis 95 Gew.-% mindestens eines cyclischen Polydimethylsiloxans, und
c) 0,1 bis 5 Gew.-% mindestens eines Siliconelastomers.
zur temporären Verformung keratinischer Fasern.

Haarverformungsmittel, die neben weiteren Bestandteilen auch Siliconelastomereenthalten, werden beispielsweise in der europaïschen Patentanmeldung EP 1 792 640 A1 beschrieben.

Vorzugsweise enthalten die kosmetischen Mittel das hydrophobierte Siliziumdioxid in einer Menge von 2 bis 12 Gew.-%, bezogen auf das gesamte Mittel, besonders bevorzugt in einer Menge von 5 bis 10 Gew.-%.

Die Herstellung hydrophobierter Siliziumdioxide ist bekannt. Eine ganze Reihe entsprechender Produkte ist kommerziell verfügbar. Die Art des eingesetzten hydrophobierten Siliciumdioxids ist nicht prinzipiell beschränkt, vorzugsweise werden jedoch hydrophobierte Siliciumdioxide eingesetzt, die durch Silanisierung von pyrogenem Siliciumdioxid erhalten werden. Man bezeichnet diese auch als trimethyliertes Silica, das unter der INCI-Bezeichnung Silica Silylate bekannt ist.

Eine Vielzahl geeigneter hydrophobierter Siliciumdioxide ist kommerziell erhältlich. Beispielhaft seien Aerosil^{®} R805, Aerosil^{®} R812, Aerosil^{®} R812S, Aerosil^{®} R816 und Aerosil^{®} R8200, alle Degussa, HDK^{®} H2000, HDK^{®} H2050 und HDK^{®} H3004, alle Wacker, sowie Dow Corning^{®} VM-2260 Aerogel Beads und Dow Corning^{®} VM-2270 Aerogel Fine Particles, alle Dow Corning genannt.

Besonders bevorzugt werden die hydrophobierten Siliciumdioxide eingesetzt, die unter den Bezeichnungen Dow Corning^{®} VM-2260 Aerogel Beads und Dow Corning^{®} VM-2270 Aerogel Fine Particles erhältlich sind. Ganz besonders bevorzugt kommt das Siliciumdioxid mit der INCI-Bezeichnung Silica Silylate zum Einsatz, das von der Firma Dow Corning unter der Bezeichnung Dow Corning^{®} VM-2260 Aerogel Beads vertrieben wird.

Vorzugsweise enthalten die kosmetischen Mittel das cyclische Polydimethylsiloxan, bezogen auf das gesamte Mittel, in einer Menge von 80 bis 95 Gew.-%.

Vorzugsweise kommen als cyclische Polydimethylsiloxane flüchtige Siloxane mit 3 bis 8 Siliziumatomen im Ring zum Einsatz. Besonders bevorzugt ist das cyclische Polydimethylsiloxan ausgewählt aus Cyclotrisiloxan, Cyclotetrasiloxan, Cyclopentasiloxan, Cyclohexasiloxan und Cycloheptasiloxan, insbesondere bevorzugt aus Cyclopentasiloxan und Cyclohexasiloxan.

Die kosmetischen Mittel enthalten das Siliconelastomer vorzugsweise in einer Menge von 0,5 bis 5 Gew.-%, bezogen auf das gesamte Mittel, besonders bevorzugt in einer Menge von 1 bis 5 Gew.-%.

Vorzugsweise handelt es sich bei dem Siliconelastomer um ein vernetztes Polysiloxan, das durch Umsetzung eines Polysiloxans, das mindestens ein direkt an ein Siliziumatom gebundenes Wasserstoffatom aufweist, mit einem alpha,omega-Alkyldien mit 5 bis 24 Kohlenstoffatomen erhalten wird. Entsprechende Siliconelastomere können beispielsweise entsprechend der Lehre der US 5,654,362 A hergestellt werden.

In einer besonders bevorzugten Ausführungsform der Erfindung wird das Siliconelastomer in Form einer Mischung aus einem vernetzten Siliconpolymer mit der INCI-Bezeichnung Dimethicone Crosspolymer und Cyclopentasiloxan eingesetzt. Entsprechende Mischungen sind kommerziell erhältlich und werden beispielsweise von Dow Corning unter der Bezeichnung Dow Corning^{®} 9040 Silicone Elastomer Blend und Dow Corning^{®} 9045 Silicone Elastomer Blend vermarktet. Der Einsatz von Dow Corning^{®} 9045 Silicone Elastomer Blend ist ganz besonders bevorzugt.

Vorzugsweise enthalten die Mittel neben den oben genannten Komponenten weiterhin mindestens ein Dimethiconol der Formeln (I) 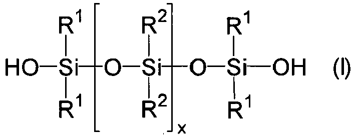 oder (II) 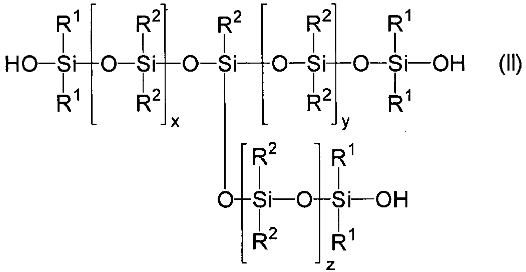 wobei in den Formeln (I) und (II)
- R¹ für Wasserstoff, einen Methylrest, einen C₂ bis C₃₀ linearen, gesättigten oder ungesättigten Kohlenwasserstoffrest, einen Phenylrest und/oder einen Arylrest,
- R² für Wasserstoff, einen Methylrest, einen C₂ bis C₃₀ linearen, gesättigten oder ungesättigten Kohlenwasserstoffrest, einen Phenylrest und/oder einen Arylrest, und
- x, y und z jeweils unabhängig voneinander für,eine ganze Zahl von 0 bis 50.000 stehen.

Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und Decyl, Alkenylreste, wie Vinyl, Halogenvinyl, Alkylvinyl, Allyl, Halogenallyl, Alkylallyl, Phenylreste und Benzylreste ein.

Vorzugsweise stehen R¹ und R² jeweils unabhängig voneinander für einen Alkylrest, der 1 bis 6 Kohlenstoffatome enthält, insbesondere bevorzugt stehen R¹ und R² für Methyl.

Vorzugsweise stehen x, y und z jeweils unabhängig voneinander für ganze Zahlen von 0 bis 10.000.

Die Molgewichte der Dimethiconole liegen vorzugsweise zwischen 1.000 D und 10.000.000 D. Die Viskositäten liegen vorzugsweise zwischen 100 und 10.000.000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1.000 und 5.000.000 cPs, ganz besonders bevorzugte Viskositäten liegen zwischen 10.000 und 3.000.000 cPs. Der bevorzugteste Bereich liegt zwischen 50.000 und 2.000.000 cPs.

Beispiele für derartige Produkte sind die folgenden Handelsprodukte: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Bevorzugt wird das Dimethiconol in Form des Handelsprodukts Dow Corning 1501 Fluid zugegeben. Das Dimethiconol liegt in diesem Fall bereits als Mischung mit Cyclopentasiloxan vor.

Das Dimethiconol kann beispielsweise in einer Menge bis 10 Gew.-%, bezogen auf das gesamte Mittel, zugegeben werden. Vorzugsweise wird das Dimethiconol in einer Menge von 1 bis 8 Gew.-%, bezogen auf das gesamte Mittel, zugegeben, besonders bevorzugt in einer Menge von 2 bis 5 Gew.-%.

Die Mittel zeichnen sich durch eine einzigartige Konsistenz aus, die in etwa der Konsistenz von Wackelpudding entspricht. Das Aussehen ähnelt einem trüben Gel, wobei die Masse jedoch wesentlich formbeständiger ist, als klassische Stylinggele. Dennoch ist die Masse flexibel und deutlich weicher als herkömmliche Haarwachse. Verreibt man die Masse zwischen den Fingern, fühlt sich diese angenehm trocken und nicht klebrig an.

Neben den genannten Komponenten können die Mittel weiterhin Parfümkomponenten und/oder Farbstoffe enthalten. Es können prinzipiell alle Parfümkomponenten und Farbstoffe eingesetzt werden, die zum Einsatz in kosmetischen Mitteln zugelassen sind. Vorzugsweise werden jedoch solche Parfümkomponenten und/oder Farbstoffe eingesetzt, die eine hinreichende Löslichkeit in cyclischen Polydimethylsiloxanen aufweisen, oder in den Mitteln gleichmäßig dispergiert werden können.

Die Mittel können allein aus den oben genannten Bestandteilen bestehen. Insbesondere können sie die Silikonbestandteile als einzige Stylingkomponente enthalten, und sind in einer bevorzugten Ausführungsform daher frei von weiteren Polymeren, insbesondere Polymeren mit Styling-Effekt, d.h. von filmbildenden und/oder festigenden Polymeren. Deren Einsatz ist allerdings nicht prinzipiell ausgeschlossen. In einer besonders bevorzugten Ausführungsform bestehen die Mittel allein aus den oben genannten Bestandteilen.

Die cyclischen Dimethicone dienen unter anderem als Träger für die weiteren Komponenten der Mittel. Auf die Zugabe weiterer Lösungsmittel kann daher verzichtet werden. Die Mittel können jedoch auch Alkohole enthalten. Ethanol und Isopropanol sind dabei bevorzugte Alkohole.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte Lösungsmittel sind Glycerin, Ethylenglykol, Propylenglykol und Phenoxyethanol in einer Menge bis 30 Gew.-% bezogen auf das gesamte Mittel.

Die Mittel können zudem die für Stylingmittel bekannten üblichen weiteren Hilfs-, Wirk- und Zusatzstoffe enthalten.

Beispielsweise können die Mittel weiterhin mindestens einen Emulgator oder ein Tensid enthalten. Dabei ist allerdings zu beachten, dass größere Mengen an Emulgatoren oder Tensiden die gewünschte Konsistenz der Mittel negativ beeinflussen können. Art und Menge dieser Bestandteile ist daher sorgfältig auszuwählen. Vorzugsweise wird auf die Zugabe von Emulgatoren und Tensiden verzichtet.

Als Emulgatoren kommen prinzipiell sowohl anionische, ampholytische, kationische und nichtionische oberflächenaktive Verbindungen in Frage, die für die Verwendung am menschlichen Körper geeignet sind. Die Gruppe der ampholytischen oberflächenaktiven Verbindungen umfasst dabei zwitterionische oberflächenaktive Verbindungen und Ampholyte. Die Verwendung von anionischen und nichtionischen oberflächenaktiven Verbindungen ist erfindungsgemäß bevorzugt.

Anionische oberflächenaktive Verbindungen sind gekennzeichnet durch eine wasserlöslichmachende anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Beispiele für geeignete anionische oberflächenaktive Verbindungen sind, jeweils in Form der Natrium-, Kalium-, Magnesium- und Ammonium- sowie der Mono-, Di- und Trialkanolammoniumsalze mit 2 oder 3 C-Atomen in der Alkanolgruppe, lineare Fettsäuren (Seifen), Ethercarbonsäuren der Formel R-O-(CH₂-CH₂O)_{X} CH₂-COOH, in der R eine lineare Alkylgruppe mit 10 bis 22 C-Atomen und x = 0 oder 1 bis 16 ist, Amidethercarboxylate der Formel [R-NH(-CH₂-CH₂O)ₙCH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht, Acylsarcoside, Acyltauride, Acylisethionate, Sulfobernsteinsäuremono- und dialkylester, Sulfobernsteinsäuremono-alkylpolyoxyethylester, lineare Alkansulfonate, lineare Alpha-Olefinsulfonate, Alpha-Sulfofettsäuremethylester, Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist, Gemische oberflächenaktiver Hydroxysulfonate, sulfatierte Hydroxyalkylpolyethylen- und/oder Hydroxyalkylenpropylenglykolether, Sulfonate ungesättigter Fettsäuren, Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, Kokosmonoglyceridsulfate, Phosphorsäuremono-, -di- und -triester von alkoxylierten Fettalkoholen und ihre Mischungen, wie beispielsweise die unter dem Warenzeichen Hostaphat^{®} vertriebenen Produkte, sowie Ester von hydroxysubstituierten Bi- oder Tricarbonsäuren mit polyhydroxylierten organischen Verbindungen, die aus der Gruppe, die veretherte (C₆-C₁₈)-Alkyl-Polysaccharide mit 1 bis 6 monomeren Saccharideinheiten und veretherte aliphatische (C₆-C₁₆)-Hydroxyalkyl-Polyole mit 2 bis 16 Hydroxylresten umfasst, ausgewählt sind und die in der Europäischen Patentschrift EP 0 258 814 B1, auf die ausdrücklich Bezug genommen wird, offenbart sind.

Bevorzugte anionische oberflächenaktive Verbindungen sind die Salze der Ethercarbonsäuren sowie phosphatgruppenhaltige Verbindungen, insbesondere die Phosphorsäuremono-, -di- und - triester von ethoxylierten C₁₀-C₁₈-, insbesondere C₁₂-C₁₄-, Fettalkoholen mit Ethoxylierungsgraden von 2 bis 10, insbesondere von 3 bis 5.

Nichtionische oberflächenaktive Verbindungen enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl.

Bevorzugte nichtionogene oberflächenaktive Verbindungen sind die Anlagerungsprodukte von Alkylenoxid, insbesondere Ethylenoxid, an Fettalkohole und Fettsäuren.

Als zwitterionische oberflächenaktive Verbindungen werden solche Substanzen bezeichnet, die im Molekül mindestens eine quartäre Ammoniumgruppe und mindestens eine -COO⁽⁻⁾ - oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche Substanzen verstanden, die außer einer C₈-C₁₈-Alkyl- oder Acylgruppe im Molekül mindestens eine freie Aminogruppe und mindestens eine -COOH- oder - SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Bevorzugte Ampholyte sind das N-Kokosalkylaminopropionat, das Kokosacylaminoethylaminopropionat und das C₁₂₋₁₈-Acylsarcosin.

Beispiele für kationische oberflächenaktive Verbindungen sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie sie beispielsweise unter dem Warenzeichen Dehyquart^{®} vertrieben werden, sowie quaternisierte Proteinhydrolysate und Silikonverbindungen erfindungsgemäß verwendet werden.

Bei den als oberflächenaktive Verbindungen eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den oberflächenaktiven Verbindungen, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden.

Werden Emulgatoren eingesetzt, kommen diese vorzugsweise in Mengen von 0,1 bis 10 Gew.-%, besonders bevorzugt in Mengen von 0,5 bis 5 und ganz besonders bevorzugt in Mengen von 0,7 bis 3 Gew.-% zum Einsatz, wobei die Mengenangaben jeweils auf das gesamte Mittel bezogen sind.

Die Mittel können weiterhin mindestens eine Ölkomponente, ausgewählt aus pflanzlichen, mineralischen und synthetischen Ölen, enthalten. Diese Ölkomponenten können insbesondere zugesetzt werden, um die Konsistenz des Mittels zu beeinflussen. Diese Ölkomponente oder Ölkomponenten sind in den Mitteln gemäß dieser Ausführungsform vorzugsweise in Mengen kleiner 15 Gew.-%, insbesondere kleiner 10 Gew.-%, bezogen auf das gesamte Mittel, enthalten.

Zu den natürlichen und synthetischen kosmetischen Ölkomponenten sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®}OE) können bevorzugt sein.
- Esteröle. Unter Esterölen sind zu verstehen die Ester von C₆ - C₃₀ - Fettsäuren mit C₂ - C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I),

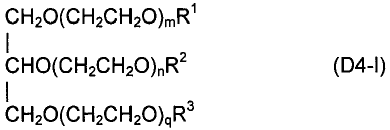

in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Obwohl nicht bevorzugt, ist auch die Zugabe filmbildender und/oder festigender Polymere möglich.

Als filmbildende und/oder festigende Polymere können alle in dieser Funktion bekannten Polymere eingesetzt werden, wobei diese sowohl permanent als auch temporär kationisch, anionisch, nichtionisch oder amphoter sein können. Bei der Verwendung von mindestens zwei filmbildenden und/oder festigenden Polymeren können diese selbstverständlich unterschiedliche Ladungen aufweisen. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft einige filmbildende Polymere beschrieben werden. Es wird an dieser Stelle jedoch explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁ bis C₇-Alkylgruppen, besonders bevorzugt C₁- bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise, unter den Handelsbezeichnungen Ucon^{®} der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird.

Festigende Polymere tragen zum Halt und/oder zum Aufbau des Haarvolumens und der Haarfülle der Gesamtfrisur bei. Diese so genannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein lang anhaltender Frisurenaufbau und -erhalt gewährleistet. Als Testmethode hierfür wird häufig der so genannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß bevorzugt zu verwendenden Polymere finden sich somit selbstverständlich gerade auch die genannten filmbildenden Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethäcrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxy-benzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Capro-Iactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate.

Bevorzugte filmbildende und/oder festigende Polymere sind die Polyvinylpyrrolidone, Vinylpyrrolidon-Vinylacetat-Copolymere, Vinylacetat-Crotonsäure-Copolymere, Vinylcaprolactam-Vinylpyrrolidon-Dimethylaminoethylmethacrylat-Copolymere, Octylacrylamid-Acrylat-Butylamino-ethyl-Methacrylat-Copolymere und quaternierte Vinylpyrrolidon-Dimethylaminoethylmethacrylat-Copolymere.

Besonders bevorzugt handelt es sich bei dem filmbildenden und/oder festigenden Polymer um die Vinylpyrrolidon-Vinylacetat-Copolymeren Luviskol^{®} VA 37 oder PVP/VA Copolymer 60/40 W NP, das Vinylacetat-Crotonsäure-Copolymer, das unter der Handelsbezeichnung Aristoflex^{®} A 60 vertrieben wird, das Vinylcaprolactam-Vinylpyrrolidon-Dimethylaminoethylmethacrylat-Copolymer mit der Handelsbezeichnung Advantage^{®} LC-E, das unter der Bezeichnung Amphomer^{®} erhältliche amphotere Octylacrylamid-Acrylat-Butylaminoethyl-Methacrylat-Copolymer oder das durch Umsetzung mit Diethylsulfat quaternierte Vinylpyrrolidon-Dimethylaminoethylmethacrylat-Copolymer, das unter der Handelsbezeichnung Gafquat^{®} 755N vertrieben wird.

Die können weiterhin die Hilfs-, Wirk- und Zusatzstoffe enthalten, die üblicherweise herkömmlichen Stylingmitteln zugesetzt werden.

Als weitere geeignete Hilfs-, Wirk- und Zusatzstoffe, die den Mitteln zugesetzt werden können, sind insbesondere Pflegestoffe zu nennen

Da die Mittel jedoch bereits verschiedene Silikone enthalten, die auch pflegende Eigenschaften besitzen, kann auf die Zugabe weiterer Pflegestoffe in der Regel verzichtet werden.

Als Pflegestoff kann beispielsweise ein von den zwingenden Inhaltsstoffen der Mittel unterschiedliches Silikonöl und/oder Silikongum eingesetzt werden. Dabei ist allerdings wiederum darauf zu achten, dass Menge und Art der Komponente so gewählt werden, dass die gewünschte Konsistenz erhalten bleibt.

Als Pflegestoff eignen sich ebenfalls pflegende Polymere, beispielsweise amphotere Polymere.

Als Pflegestoff kann weiterhin mindestens ein Vitamin, ein Provitamin, eine Vitaminvorstufe und/oder eines derer Derivate eingesetzt werden.

Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin B₅ (Pantothensäure, Panthenol und Pantolacton).

Als Pflegestoff kann weiterhin mindestens ein Pflanzenextrakt eingesetzt werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Seerose, Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Als Pflegestoff eignet sich weiterhin eine Reihe von Carbonsäuren.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12C - Atomen in der Kette.

Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten, bevorzugt ist. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Neben den Pflegestoffen können auch weitere Hilfs-, Wirk- und Zusatzstoffe zugegeben werden.

Durch Zugabe eines UV-Filters können sowohl die Mittel selbst, als auch die behandelten Fasern vor schädlichen Einflüssen von UV-Strahlung geschützt werden. Es kann daher vorteilhaft sein, den Haarfestigungsmitteln mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Beispielhaft sei hier 2-Hydroxy-4-methoxybenzophenon-5-sulfonsäure und deren Natriumsalz (Benzophenone-4; Uvinul^{®}MS 40; Uvasorb^{®}S 5) genannt.

### Beispiele

Die im Folgenden angegebenen Mengenangaben verstehen sich - soweit nichts anderes vermerkt ist - in Gewichtsprozent.

### 1 Stylingmittel

Es wurden die Stylingmittel E1 bis E3 gemäß folgender Tabelle hergestellt:

| **Rohstoffe** | **E1** | **E2** | **E3** |
|---|---|---|---|
| Dow Corning^{®} 345 Fluid¹ | ad 100 | ad 100 | ad 100 |
| Dow Corning² 1501 Fluid² | 20,0 | 20,0 | - |
| Dow Corning^{®} 9040³ | - | - | 35,0 |
| Dow Corning^{®} 9045⁴ | 10,0 | 15,0 | - |
| Dow Corning^{®}VM-2260 Aerogel Beads⁵ | 8,5 | 7,5 | 9,0 |
| Parfüm | 0,015 | 0,20 | 1,00 |
| Farbstoff | 0,0003 | 0,0003 | - |

| | | | |
|---|---|---|---|
| ¹ Mischung aus Cyclopentasiloxan und Cyclohexasiloxan (INCI-Bezeichnung: Cyclopentasiloxane and Cyclohexasiloxane) (Dow Corning) ² Dimethylpolysiloxanol in Cyclopentasiloxan (ca. 15 % Festkörper; INCI-Bezeichnung: Cyclopentasiloxane and Dimethiconol) (Dow Corning) ³ Vernetztes Dimethicon (Siliconelastomer) in Cyclopentasiloxan (ca. 12,5 % Festkörper; INCI-Bezeichnung: Cyclopentasiloxane and Dimethicone Crosspolymer) (Dow Corning) ⁴ Vernetztes Dimethicon (Siliconelastomer) in Cyclopentasiloxan (ca. 12,5 % Festkörper; INCI-Bezeichnung: Cyclopentasiloxane and Dimethicone Crosspolymer) (Dow Corning) ⁵ Trimethyliertes Silica (INCI-Bezeichnung: Silica Silylate) (Dow Corning) | | | |

Zur Herstellung der Stylingmittel E1 und E2 wurde zunächst Dow Corning^{®} 1501 Fluid vorgelegt und unter langsamem Rühren das Siliconelastomer Dow Corning^{®} 9045 zugegeben. Nach circa fünfminütigem Rühren war eine sirupartige Masse entstanden, zu der unter langsamem Rühren portionsweise Dow Corning^{®} 345 Fluid zugegeben wurde. Der Farbstoff wurde in dem Parfüm gelöst und die Lösung anschließend unter Rühren der Mischung der Silikonkomponenten zugegeben. Das Rühren wurde unterbrochen und der Masse die gesamte Menge Dow Corning^{®} VM-2260 Aerogel Beads zugegeben. Es wurde wieder gerührt bis eine homogene Masse entstanden war.

Zur Herstellung des Stylingmittels E3 wurde das Siliconelastomer Dow Corning^{®} 9045 vorgelegt und unter langsamem Rühren portionsweise Dow Corning^{®} 345 Fluid zugegeben. Das Rühren wurde unterbrochen und der Mischung die gesamte Menge Dow Corning^{®} VM-2260 Aerogel Beads zugegeben. Es wurde wieder gerührt bis eine homogene Masse entstanden war.

Die Stylingmittel zeichnen sich durch eine einzigartige Konsistenz aus, die an Wackelpudding erinnert. Die Masse ist flexibel und hinterlässt auf der Haut ein angenehm trockenes und nicht klebriges Gefühl.

## Patentansprüche

1. Verwendung eines kosmetischen Mittels, enthaltend, jeweils bezogen auf das gesamte Mittel,
a) 2 bis 15 Gew.-% mindestens eines hydrophobierten Siliziumdioxids,
b) 50 bis 95 Gew.-% mindestens eines cyclischen Polydimethylsiloxans, und
c) 0,1 bis 5 Gew.% mindestens eines Siliconelastomers.
zur temporären Verformung keratinischer Fasern.

2. Verwendung gemäß Anspruch 1, **dadurch gekennzeichnet, dass** es das hydrophobierte Siliziumdioxid in einer Menge von 2 bis 12 Gew.-%, bezogen auf das gesamte Mittel, enthält.

3. Verwendung gemäß wenigstens eines der Ansprüche 1 und 2, **dadurch gekennzeichnet, dass** es sich bei dem hydrophobierten Siliziumdioxid um silanisiertes pyrogenes Siliziumdioxid handelt.

4. Verwendung gemäß wenigstens eines der Ansprüche 1 bis 3, **dadurch gekennzeichnet, dass** das cyclische Polydimethylsiloxan ausgewählt ist aus Cyclotrisiloxan, Cyclotetrasiloxan, Cyclopentasiloxan, Cyclohexasiloxan und Cycloheptasiloxan.

5. Verwendung gemäß Anspruch 4, **dadurch gekennzeichnet, dass** das cyclische Polydimethylsiloxan ausgewählt ist aus Cyclopentasiloxan und Cyclohexasiloxan.

6. Verwendung gemäß wenigstens eines der Ansprüche 1 bis 5, **dadurch gekennzeichnet, dass** es sich bei dem Siliconelastomer um ein vernetztes Polysiloxan handelt, das durch Umsetzung eines Polysiloxans, das mindestens ein direkt an ein Siliziumatom gebundenes Wasserstoffatom aufweist, mit einem alpha,omega-Alkyldien mit 5 bis 24 Kohlenstoffatomen erhalten wird.

7. Verwendung gemäß wenigstens eines der Ansprüche 1 bis 6, **dadurch gekennzeichnet, dass** es weiterhin mindestens ein Dimethiconol der Formeln (I) 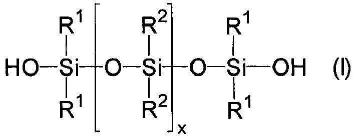 oder (II) 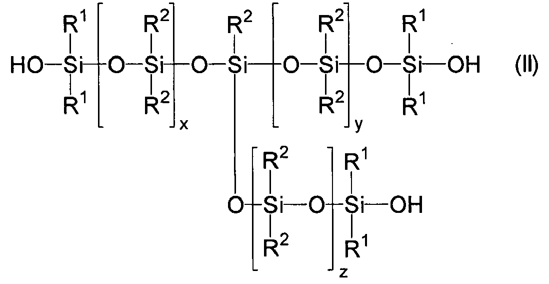 in einer Menge von 0 bis 10 Gew.-%, bezogen auf das gesamte Mittel, enthält, wobei in den Formeln (I) und (II)
- R¹ für Wasserstoff, einen Methylrest, einen C₂ bis C₃₀ linearen, gesättigten oder ungesättigten Kohlenwasserstoffrest, einen Phenylrest und/oder einen Arylrest,
- R² für Wasserstoff, einen Methylrest, einen C₂ bis C₃₀ linearen, gesättigten oder ungesättigten Kohlenwasserstoffrest, einen Phenylrest und/oder einen Arylrest, und
- x, y und z jeweils unabhängig voneinander für eine ganze Zahl von 0 bis 50.000 stehen.

8. Verwendung gemäß Anspruch 7, **dadurch gekennzeichnet, dass** es das Dimethiconol der Formeln (I) oder (II) in einer Menge von 1 bis 8 Gew.-%, bezogen auf das gesamte Mittel, enthält.

## Claims

1. The use of a cosmetic agent which contains, in each case based on the total agent,
a) 2 to 15 % by weight of at least one hydrophobized silicon dioxide,
b) 50 to 95 % by weight of at least one cyclic polydimethylsiloxane, and
c) 0.1 to 5 % by weight of at least one silicone elastomer,
for temporarily deforming keratin fibers.

2. The use according to claim 1, **characterized in that** the agent contains the hydrophobized silicon dioxide in an amount of from 2 to 12 % by weight based on the total agent.

3. The use according to at least one of claims 1 and 2, **characterized in that** the hydrophobized silicon dioxide is a silanized pyrogenic silicon dioxide.

4. The use according to at least one of claims 1 to 3, **characterized in that** the cyclic polydimethylsiloxane is selected from cyclotrisiloxane, cyclotetrasiloxane, cyclopentasiloxane, cyclohexasiloxane and cycloheptasiloxane.

5. The use according to claim 4, **characterized in that** the cyclic polydimethylsiloxane is selected from cyclopentasiloxane and cyclohexasiloxane.

6. The use according to at least one of claims 1 to 5, **characterized in that** the silicone elastomer is a cross-linked polysiloxane which is obtained by reacting a polysiloxane comprising at least one hydrogen atom which is directly bonded to a silicon atom with an alpha, omega-alkyldiene having 5 to 24 carbon atoms.

7. The use according to at least one of claims 1 to 6, **characterized in that** the agent also comprises at least one dimethiconol of formula (I) 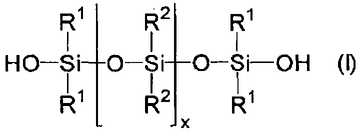 or formula (II) 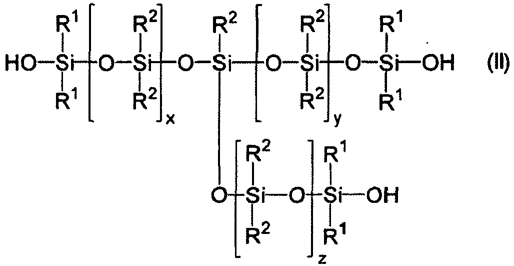 in an amount of from 0 to 10 % by weight, based on the total agent, where, in formulas (I) and (II),
- R¹ represents hydrogen, a methyl functional group, a C₂ to C₃₀ linear, saturated or unsaturated hydrocarbon functional group, a phenyl functional group and/or an aryl functional group,
- R² represents hydrogen, a methyl functional group, a C₂ to C₃₀ linear, saturated or unsaturated hydrocarbon functional group, a phenyl functional group and/or an aryl functional group, and
- x, y and z each represent, independently of one another, an integer of from 0 to 50,000.

8. The use according to claim 7, **characterized in that** the agent contains the dimethiconol of formula (I) or formula (II) in an amount of from 1 to 8 % by weight, based on the total agent.

## Revendications

1. Utilisation d'un agent cosmétique contenant, à chaque fois par rapport à tout l'agent,
a) de 2 à 15 % en poids d'au moins un dioxyde de silicium hydrophobe,
b) de 50 à 95 % en poids d'au moins un polydiméthylsiloxane cyclique, et
c) de 0,1 à 5 % d'au moins un élastomère de silicone,
pour la mise en forme temporaire de fibres de kératine.

2. Utilisation selon la revendication 1, **caractérisée en ce qu'**il contient le dioxyde de silicium hydrophobe dans une quantité de 2 à 12 % en poids, par rapport à tout l'agent.

3. Utilisation selon au moins une des revendications 1 et 2, **caractérisée en ce que** le dioxyde de silicium hydrophobisé est du dioxyde de silicium pyrogéné silané.

4. Utilisation selon au moins une des revendications 1 à 3, **caractérisée en ce que** le polydiméthylsiloxane cyclique est choisi parmi le cyclotrisiloxane, le cyclotétrasiloxane, le cyclopentasiloxane, le cyclohexasiloxane et le cycloheptasiloxane.

5. Utilisation selon la revendication 4, **caractérisée en ce que** le polydiméthylsiloxane cyclique est choisi parmi le cyclopentasiloxane et le cyclohexasiloxane.

6. Utilisation selon au moins l'une des revendications 1 à 5, **caractérisée en ce que** l'élastomère de silicone est un polysiloxane réticulé qui est obtenu par réaction d'un polysiloxane, qui comporte au moins un atome d'hydrogène directement lié à un atome de silicium, avec un alpha, omega-alkylidène ayant 5 à 24 atomes de carbone.

7. Utilisation selon au moins l'une des revendications 1 à 6, **caractérisée en ce qu'**il contient en outre au moins un diméthiconol de la formule (I) 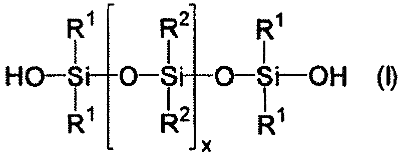 ou (II) 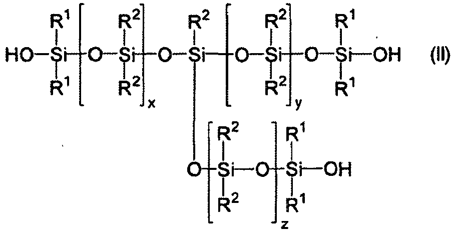 dans une quantité de 0 à 10 % en poids, par rapport à tout l'agent, dans les formules (I) et (II)
- R¹ représentant l'hydrogène, un radical méthyle, un radical hydrocarbure en C₂ à C₃₀ linéaire, saturé ou insaturé, un radical phényle et/ou un radical aryle,
- R² représentant un atome d'hydrogène, un radical méthyle, un radical hydrocarbure en C₂ à C₃₀ linéaire, saturé ou insaturé, un radical phényle et/ou un radical aryle, et
- x, y et z représentant chacun indépendamment un nombre entier de 0 à 50 000.

8. Utilisation selon la revendication 7, **caractérisée en ce qu'**elle contient le diméthiconol des formules (I) ou (II) dans une quantité de 1 à 8 % en poids, par rapport à tout l'agent.
